# EUROPÄISCHE PATENTANMELDUNG

(11) **EP 4 574 161 A1**
(43) Veröffentlichungstag der Anmeldung: **25.06.2025**
(21) Anmeldenummer: 24170577.1
(22) Anmeldetag: 16.04.2024
(51) Int. Cl.: A61K 38/01, A61K 38/39, A23L 33/10, A23L 33/18, A61K 9/00, A61K 9/08, A61K 9/10, A61K 31/19, A61P 5/00, A61P 7/08, A61P 13/12

(54) **ZUSAMMENSETZUNG ZUR EIWEISSSUBSTITUTION**

(30) Priorität: 22.12.2023 DE 202023107635 U
(71) Anmelder: RenaCare NEPHROMED GmbH, 35625 Hüttenberg (DE)
(72) Erfinder: BARTZ, Katharina, 35440 Linden (DE); LESCH, Lisa, 35440 Linden (DE)
(74) Vertreter: Rommeswinkel, Marike

(57) **Zusammenfassung**

Die vorliegende Erfindung betrifft eine Zusammensetzung, insbesondere zur Eiweißsubstitution bei Niereninsuffizienz, welche in flüssiger Form vorliegt und Kollagen-Hydrolysate in einer Menge von 20 bis 50 Gew.-%, und 3-Hydroxy-3-methylbutter-säure in einer Menge von 0,1 bis 5 Gew.-%, jeweils bezogen auf die Zusammensetzung, enthält. Weiterhin betrifft die Erfindung die Verwendungen der Zusammensetzung.

## Beschreibung

Die vorliegende Erfindung betrifft das technische Gebiet der Nahrungsergänzungsmittel bzw. Lebensmittel für besondere medizinische Zwecke, insbesondere zur therapeutischen Behandlung von Erkrankungen bzw. für eine bilanzierte Diät.

Insbesondere betrifft die vorliegende Erfindung eine Zusammensetzung, welche sich zur Eiweißsubstitution bei Niereninsuffizienz, insbesondere von Dialysepatienten, eignet.

Weiterhin betrifft die vorliegende Erfindung eine Zusammensetzung zur Verwendung bei der therapeutischen Behandlung Eiweißmangelerkrankungen, der Hyperphosphatämie sowie des sekundären Hyperparathyreoidismus.

Weiterhin betrifft die vorliegende Erfindung die Verwendung einer Zusammensetzung als Nahrungsergänzungsmittel bzw. zur Substitution des erhöhten Eiweißbedarfs bei Niereninsuffizienz.

Schließlich betrifft die vorliegende Erfindung die Verwendung einer Zusammensetzung zur therapeutischen Behandlung von Eiweißmangelerkrankungen, der Hyperphosphatämie sowie des sekundären Hyperparathyreoidismus.

Der menschliche Organismus scheidet üblicherweise im Blut vorhandene und durch Stoffwechselprozesse entstehende Abfallprodukte sowie überschüssige Mengen an Elektrolyten oder Mineralstoffe über die Nieren aus. Bei einer Vielzahl von Menschen ist diese Ausscheidung von Abfallprodukten bzw. der überschüssigen Elektrolyten und Mineralstoffen jedoch gestört. Führt die Funktionsstörung der Niere dazu, dass die Abfallprodukte bzw. überzählige Elektrolyte und Mineralstoffe nicht in ausreichendem Maße ausgeschieden werden, so spricht man von einer Niereninsuffizienz, welche je nach Ausprägung mit einem erheblichen Verlust an Lebensqualität bzw. mit einer Gefahr für Leben und Gesundheit einhergeht. Kommt die Nierenfunktion vollständig oder nahezu vollständig zum Erliegen, so liegt eine terminale Insuffizienz vor und der Patient ist auf eine apparative Entfernung der unerwünschten Stoffe aus dem Blutkreislauf als Nierenersatztherapie angewiesen. Ein Großteil der Patienten mit Nierenversagen erhält eine Hämodialyse. Ein kleiner Teil der Patienten wird mit einer Peritioneal- oder Bauchfelldialyse behandelt.

In Deutschland werden derzeit ca. 80.000 Dialysepatienten behandelt. Der Großteil dieser Patienten besitzt keine Restausscheidung an Urin mehr oder kann nur noch kleine Mengen Resturin ohne nennenswerte Konzentrationsfähigkeit produzieren. Das bedeutet, dass die durch die körperlichen Stoffwechselprozesse entstehenden Abfallprodukte nicht mehr ausgeschieden werden können und lediglich eine Wasserausscheidung stattfindet, welche jedoch oftmals unzureichend ist, falls überhaupt vorhanden.

Neben den Abfallprodukten und dem Überschuss an Wasser können die Nieren auch ein Zuviel an Elektrolyten oder Mineralstoffen nicht mehr ausscheiden. Dies ist besonders im Hinblick auf die im Blut enthaltenen an sich essentiellen Bestandteile Phosphat und Kalium besonders kritisch: So führt eine zu hohe Konzentration an Kalium, die sogenannte Hyperkaliämie, zu einem Herzstillstand, während ein zu hoher Phosphatspiegel im Blut, die sogenannte Hyperphosphatämie, oftmals zum sekundären Hyperparathyreoidismus führt.

Da die natürliche Homöostase im Blut, d. h. der Gleichgewichtszustand des natürlichen inneren Milieus durch die mangelnde Ausscheidung gestört ist, unterziehen sich Dialysepatienten üblicherweise dreimal in der Woche einer Dialyse, bei welcher die Konzentration der einzelnen Blutbestandteile wieder dem Sollzustand, d. h. den im Blut von Gesunden vorliegenden Konzentrationen, angeglichen wird.

Die Dialyse führt jedoch dazu, dass Dialysepatienten einen erhöhten Eiweiß- bzw. Proteinbedarf besitzen: Während der Proteinbedarf in der Normalbevölkerung bei ca. 0,8 g/kg Körpergewicht und Tag liegt, wird der Proteinbedarf für Dialysepatienten in der Literatur übereinstimmend mit 1,2 g/kg Körpergewicht und Tag angegeben, d. h. Dialysepatienten benötigen ca. 50 % mehr Eiweiß bzw. Protein als die Normalbevölkerung. Eiweiße sind wichtige Bestandteile der Körperzellen und erfüllen im Blut als Hormone, wie beispielsweise Insulin oder Glucagon, oder als Transportmittel, wie beispielsweise Erythrozyten oder Albumin, eine wichtige Aufgabe.

Der erhöhte Eiweißbedarf ist dem Verlust an Protein bei der Dialyse geschuldet. So wird beispielsweise ein Teil der Eiweiße, wie beispielsweise die roten Blutkörperchen oder Albumin, durch Zusammenstöße mit den Oberflächenstrukturen der Plastikschläuche und des Dialysators zerstört. Weitere Eiweiße werden in der Blutpumpe zerkleinert bzw. frakturiert, was sich neben dem Funktionsverlust der Eiweiße vor allen Dingen darin äußert, dass kleine Eiweiße, insbesondere kurze Aminosäuresequenzen - die sogenannten Peptide -, konzentrationsabhängig aus dem Blut entfernt werden, da der Dialysator nach dem Prinzip der Osmose arbeitet. Schließlich verbleibt darüber hinaus immer eine kleine Restmenge Blut im Dialysesystem, womit ein weiterer Eiweißverlust verbunden ist. Um diesen ständigen Eiweißverlust auszugleichen, müssen Dialysepatienten eine größere Menge an Eiweiß zu sich nehmen.

Problematisch hierbei ist jedoch, dass Eiweiß aus natürlichen Quellen, wie es beispielsweise in pflanzlichen oder tierische Produkten enthalten ist, üblicherweise nur in Verbund mit Phosphat auftritt, d. h. dass das Phosphat an die Eiweiße gebunden ist. Eine zu hohe Konzentration von Phosphaten im Blut, die sogenannte Hyperphosphatämie, führt jedoch zu Folgeerkrankungen und stellt das größte Problem chronisch nierenkranker Patienten dar. Insbesondere Dialysepatienten sind betroffen, da sie - wie zuvor bereits ausgeführt - im Vergleich zur Normalbevölkerung einen Proteinmehrbedarf von ca. 50 % besitzen. Dies bedeutet, dass das bei einer ausreichenden Eiweißaufnahme die Dialysepatienten auch vermehrt Phosphat aufnehmen und zwar ebenfalls ca. 50 % mehr als die Normalbevölkerung.

Gesunde scheiden einen Überschuss an Phosphat, wie er nach dem Verzehr phosphathaltiger Nahrungsmittel - beispielsweise Cola - auftreten kann, anhand des homöostatischen Prinzips über die Nieren wieder aus. Dieser Weg steht Patienten mit Niereninsuffizienz und insbesondere Dialysepatienten nicht zur Verfügung. Sie können das freie Phosphat nur über eine Bindung an Calcium inaktivieren, wobei jedoch der Calciumspiegel im Blutserum sinkt. Der verminderte Calciumspiegel im Blutserum führt jedoch zu einer Aktivierung bzw. einer vermehrten Bildung von Parathormon in der Nebenschilddrüse, dem sogenannten sekundären Hyperparathyreoidismus. Durch Parathormon werden die körpereigenen Calciumreserven aus Knochen und Zähnen mobilisiert, so dass Calciumionen im Blutkreislauf für die Komplexbildung bzw. Ausfällung des Phosphats zur Verfügung stehen. Durch diesen fortlaufenden Prozess kommt es zu einer Osteopathie und Osteomalazie, d. h. einer Verweichlichung der Knochen, mit verminderter Knochenmasse sowie einer Verschlechterung der Mikroarchitektur des Knochens mit entsprechend reduzierter Festigkeit.

Für den Organismus ist jedoch nicht nur der fortwährende Entzug von Calcium aus Knochen und Zähnen problematisch, sondern auch die sich bildenden Phosphat-Calcium-Komplexe. Die Phosphat-Calcium-Komplexe werden im Körper in den Weichteilen eingelagert - wo sie zur Juckreiz führen - und darüber hinaus auch in den Arterien. Durch diese Art von Arteriosklerose kommt es regelmäßig zu Herzinfarkten und Schlaganfällen. Verstärkt wird dieser Prozess bei Patienten mit chronischer Niereninsuffizienz, insbesondere bei Dialysepatienten, dadurch, dass der Gegenspieler des Parathormons, das Vitamin D, unter Sonneneinstrahlung in der Haut gebildet wird und anschließend in der Leber und den Nieren aktiviert werden muss. Gerade diese Aktivierung findet bei Patienten mit chronischer Niereninsuffizienz jedoch nicht oder nur noch in untergeordnetem Maße statt.

Durch die Dialyse wird zwar ein Teil des Phosphats aus dem Blut entfernt, dies ist jedoch nicht ausreichend, wie am nachfolgenden Beispiel verdeutlicht werden soll: So müsste ein 70 kg schwerer Dialysepatient gemäß den Ernährungsempfehlungen 84 g Eiweiß pro Tag zu sich nehmen. Die Eiweißquellen enthalten im Durchschnitt 17 mg Phosphat pro Gramm Eiweiß, um als nierengesundes Eiweiß geführt zu werden. Dieser Wert ist beispielsweise mit einer Vielzahl von Milchprodukten nicht einzuhalten. Selbst wenn pro Gramm Eiweiß nur 17 mg Phosphat an das Eiweiß gebunden sind, würde der Patient immer noch ca. 1.428 mg Phosphat pro Tag und ca. 10.000 mg Phosphat in der Woche zu sich nehmen, wovon etwa 60 bis 70 % vom Körper resorbiert werden. Durch Dialyse können jedoch in einer Sitzung nur 800 bis 900 mg Phosphat entfernt werden, d. h. bei drei Anwendungen in der Woche können 2.400 mg bis 2. 700 mg Phosphat aus dem Blut entfernt werden. Dies ist jedoch bei Weitem nicht ausreichend, so dass in der Praxis Phosphatbinder gegeben werden, welche bei richtiger zielgerichteter Einnahme die Phosphatresorption auf ca. 40 bis 50 % beschränken können. Aber auch unter Berücksichtigung dieser Maßnahmen verbleibt ein Überschuss von ca. 1.400 bis 2.200 mg Phosphat in der Woche, welcher nicht entfernt werden kann. Aus diesem Grund muss die Diät der Dialysepatienten so beschaffen sein, dass die tägliche Phosphatzufuhr auf ca. 1.000 mg pro Tag begrenzt ist.

Da es schwierig ist, diese Diät dauerhaft mit natürlichen Produkten einzuhalten, ohne dass ein Eiweißmangel auftritt, werden speziell für Dialysepatienten phosphatarme Eiweißpräparate angeboten. Diese Produkte werden als Pulver vertrieben und basieren üblicherweise auf Molkeneiweißisolaten. Der Patient muss das Pulver dosieren, mit etwas Wasser anrühren oder aufschütteln und kann anschließend die Mischung als Getränk zu sich nehmen. Diese Art der Eiweißsubstitution ist prinzipiell geeignet, Dialysepatienten mit phosphatarmen Eiweißen zu versorgen, stößt jedoch in der Praxis auf eine Vielzahl von Problemen und Hindernissen.

So können beispielsweise Patienten mit einer Molkenallergie die Produkte nicht zu sich nehmen. Darüber hinaus sind die meisten Dialysepatienten über 70 Jahre alt und körperlich nicht mehr in der Lage das Pulver selbstständig aufzulösen, so dass sie auf fremde Hilfe angewiesen sind. Kann eine solche Hilfe nicht zur Verfügung gestellt werden, kann das Eiweißsubstitut aus Gründen der Compliance nicht verordnet werden. Darüber hinaus ist durch die ungenaue Dosierung mittels Messlöffeln die Gefahr von Fehldosierungen gegeben.

Neben den reinen Eiweißpräparaten auf Molkenbasis gibt es noch eine Reihe von hochkalorischen Kombinationspräparaten, bei welchen es sich um eine vollwertige Ernährung handelt. Dies bedeutet, dass neben den Molkeneiweißen zusätzlich noch Energieträger in Form von Kohlenhydraten bzw. Fetten im Ernährungsprodukt enthalten sind. Dieser zusätzliche physiologische Brennwert wirft jedoch erneute Probleme auf. Zum Einen leiden einige Dialysepatienten an reinem Eiweißmangel, d. h. in diesem Fall wäre es vollkommen unnötig, dieses Patientenkollektiv mit zusätzlichen Kalorien zu belasten; zum anderen sind fast 50 % der Dialysepatienten Diabetiker.

Beim Diabetes mellitus handelt es sich um eine Stoffwechselkrankheit auf der Grundlage eines absoluten oder relativen Insulinmangels mit daraus resultierenden hohen Zuckerkonzentrationen im Blut, der sogenannten Hyperglykämie. Diese fördert die Schädigung von Blutgefäßen und stellt damit einen weiteren Triggerfaktor für Herzinfarkte und Schlaganfälle dar. Darüber hinaus führen hohe Glukosespiegel im Blut zu Nierenschäden und können somit eine gegebenenfalls noch vorhandene Restdiurese verringern. Weitere Folgen können Nervenschäden (Polyneuropathie), Visusverlust oder ein diabetischer Fuß mit möglicher Amputation sein. Um diese Folgeerkrankungen möglichst gering zu halten, müssen Diabetiker ein strenges Ernährungsschema mit regelmäßigen Messungen des Glukosegehalts im Blut befolgen. Ein Ernährungsprodukt mit hoher Glukosedichte ist daher für Diabetiker kontrainduziert.

Darüber hinaus gibt es auch pulverförmige Eiweißpräparate auf Basis von Kollagen-Hydrolysaten, welche im Bereich der Medizin bislang nahezu ausschließlich Anwendung zur Prävention und Behandlung von Osteoporose finden.

So beschreibt die DE 10 2012 110 612 A1 ein Kollagen-Hydrolysat, welches durch enzymatische Hydrolyse von Knochengelatine vom Typ B hergestellt ist, wobei das Knochenhydrolysat aus Peptiden gebildet ist, von denen mindestens 50 Gew.-% ein Molekulargewicht von 1.500 bis 13.500 g/mol aufweisen und deren mittleres Molekulargewicht im Bereich von 4.000 bis 8.000 g/mol liegt. Das Kollagen-Hydrolysat wird als Wirkstoff zur Erhaltung bzw. Verbesserung der Gesundheit der Knochen, insbesondere zur Vorbeugung und/oder Behandlung von Osteoporose verwendet. Die vorgeschlagenen Dosierungen sind jedoch nicht für Dialysepatienten geeignet. Darüber hinaus ist nur eine schlechte Resorption der Peptidmoleküle im Körper gegeben.

Ein weiteres schwerwiegendes Problem der Niereninsuffizienz und speziell der Dialyse ist der bei den Betroffenen auftretende Skelettmuskelschwund in Kombination mit einem Verlust an Muskelmasse, dem sogenannten "protein-wasting". Um den Muskelschwund und Proteinverlust aufzuhalten oder zumindest zu verzögern, kann den Patienten eine höhere Dosis an Leucin verabreicht werden. Leucin ist eine essentielle Aminosäure und spielt eine wichtige Rolle beim Muskelaufbau und -erhalt sowie bei Heilungsprozessen. Unglücklicherweise hat Leucin einen stark sauren und unangenehmen Geschmack, weshalb stark mit Leucin angereicherte Nahrungsergänzugsmittel nur schlecht von den Dialyspatienten angenommen werden.

Die DE 20 2014 105 602 offenbart eine Eiweißzusammensetzung auf Basis von Kollagen-Hydrolysat, welche phospahtarm, gut verträglich und resorbierbar ist. Allerdings kann auch diese Zusammensetzung dem niereninsuffienz- bzw. dialysebedingten Muskelproteinverlust und Muskelschwund nur bedingt entgegenwirken.

Der vorliegenden Erfindung liegt daher die Aufgabe zugrunde, eine Zusammensetzung bereitzustellen, welche sich als Nahrungsergänzung, insbesondere zur Eiweißsubstitution bei Patienten mit chronischer Niereninsuffizienz, eignet, wobei die zuvor geschilderten Nachteile des Standes der Technik zumindest weitgehend vermieden oder aber wenigstens abgeschwächt werden sollen.

Insbesondere liegt der vorliegenden Erfindung die Aufgabe zugrunde, eine Zusammensetzung bereitzustellen, welche eine zuverlässige Versorgung von Patienten, insbesondere von Dialysepatienten, mit phosphatarmem Eiweiß sicherstellt und darüber hinaus einfach zu handhaben ist. Die Zusammensetzung soll darüber hinaus von einem möglichst großen Patientenkollektiv genutzt werden können, d. h. sie soll auch Patienten mit weiteren Erkrankungen, wie beispielsweise Diabetikern, zur Verfügung stehen.

Eine weitere Aufgabe der vorliegenden Erfindung ist darin zu sehen, eine Zusammensetzung bereitzustellen, welche den Muskelproteinverlust und Muskelschwund bei Niereninsuffizienz, insbesondere von Dialysepatienten, aufhält oder zumindest verlangsamt.

Zur Lösung der zuvor gestellten Aufgabe schlägt die vorliegende Erfindung eine Zusammensetzung gemäß Anspruch 1 vor; weitere vorteilhafte Ausgestaltungen sind Gegenstand der diesbezüglichen abhängigen Ansprüche.

Wiederum weiterer Gegenstand der vorliegenden Erfindung ist die erfindungsgemäße Zusammensetzung zur Verwendung in therapeutischen Behandlungen; weitere vorteilhafte Ausgestaltungen sind Gegenstand der diesbezüglichen abhängigen Ansprüche.

Wiederum weiterer Gegenstand der vorliegenden Erfindung ist die Verwendung der Zusammensetzung gemäß dem diesbezüglichen unabhängigen Anspruch.

Schließlich ist wiederum weiterer Gegenstand der vorliegenden Erfindung die Verwendung der Zusammensetzung in therapeutischen Behandlungen.

Es versteht sich von selbst, dass im Folgenden besondere Ausgestaltungen, Ausführungsformen oder dergleichen, welche nur in Bezug auf einen Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.

Zudem gilt, dass alle im Folgenden genannten Parameterangaben oder dergleichen grundsätzlich mit genormten oder explizit angegeben Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Aspekt der vorliegenden Erfindung - eine Zusammensetzung, insbesondere zur Eiweißsubstitution bei Niereninsuffizienz, wobei die Zusammensetzung in flüssiger Form vorliegt und
(a) Kollagen-Hydrolysate in einer Menge von 20 bis 50 Gew.-%, und
(b) 3-Hydroxy-3-methylbuttersäure in einer Menge von 0,1 bis 5 Gew.-%,
jeweils bezogen auf die Zusammensetzung, enthält.

Wie die Anmelderin herausgefunden hat, lässt sich durch die erfindungsgemäße Kombination von Kollagen-Hydrolysaten und 3-Hydroxy-3-methylbuttersäure ein hochkonzentriertes Eiweißpräparat erhalten, welches durch die 3-Hydroxy-3-methyl-buttersäure einen nochmals verbeserten Effekt im Hinblick auf den Muskelaufbau bzw. -erhalt aufweist. Insbesondere kann durch die Zugabe von 3-Hydroxy-3-methyl-buttersäure auf eine weitere Anreicherung der Zusammensetzung mit Leucin verzichtet werden. Leucin ist von besonderer Bedeutung für den Muskelaufbau, verfügt jedoch über einen stark sauren und unangenehmen Eigengeschmack. Duch die Verwendung von 3-Hydroxy-3-methylbuttersäure kann die positive Wirkung von Leucin ohne den nachteiligen Geschmack erzielt werden, wodurch die Compliance und Akzeptanz bei den Patienten deutlich verbessert wird.

3-Hydroxy-3-methylbuttersäure, auch unter den Bezeichnungen beta-Hydroxy-betamethyl-butyrat, beta-Hydroxyisovaleriansäure oder kurz HMB, bekannt, ist ein Metabolit der essentiellen Aminsäure Leucin. Leucin ist wichtig für den Erhalt und Aufbau von Muskelgewebe und unterstützt Heilungsprozesse. Eine hohe Leucinzufuhr ist daher für Patienten mit Niereninsuffizienz, insbesondere für Dialysepatienten, besonders wichtig. Für die positiven Wirkungen des Leucins ist insbesondere dessen Metabolit 3-Hydroxy-3-methylbuttersäure verantwortlich.

Im Rahmen der vorliegenden Erfindung werden besonders gute Ergebnisse erhalten, wenn die Zusammensetzung 3-Hydroxy-3-methylbuttersäure in einer Menge von 0,2 bis 3 Gew.-%, insbeondere 0,3 bis 2 Gew.-%, vorzugsweise 0,4 bis 1,5 Gew.-%, bevorzugt 0,4 bis 1 Gew.-%, bezogen auf die Zusammensetzung, enthält.

Gemäß einer speziellen Ausführungsform der vorliegenden Erfindung enthält die Zusammensetzung 3-Hydroxy-3-methylbuttersäure in Form von Calcium-3-Hydroxy-3-methylbutyrat (Ca-HMB).

Darüber hinaus ist die erfindungsgemäße Zusammensetzung besonders phosphatarm bzw. nahezu phosphatfrei und einfach zu handhaben. Die erfindungsgemäße Zusammensetzung eignet sich somit in hervorragender Weise zur Nahrungsergänzung, insbesondere zur Eiweißsubstitution bei Niereninsuffizienz und speziell zur Deckung des erhöhten Eiweißbedarfs von Dialysepatienten.

Eine weitere essentielle Komponente der erfindungsgemäßen Zusammensetzung ist Kollagen-Hydrolysat. Bei Kollagen handelt es sich um ein extrazelluläres Strukturprotein, welches etwa 20 bis 30 % des Gesamteiweißes von Tieren und Menschen ausmacht. Kollagen findet sich hauptsächlich in Bindegewebe und ist einerseits für die Festigkeit und andererseits für die Flexibilität des Bindegewebes verantwortlich.

Kollagen ist nicht wasserlöslich, kann jedoch durch Einwirkung von Wärme, Basen oder Säuren hydrolysiert werden, wodurch Gelatine erhalten wird. Im Gegensatz zu Kollagen ist Gelatine unter Wärmezufuhr in Wasser löslich bzw. dispergierbar und ist in der Lage, große Mengen Wasser zu binden.

Bei Kollagen-Hydrolysaten, wie sie im Rahmen der vorliegenden Erfindung Verwendung finden, handelt es sich jedoch nicht um Gelatine bzw. Gelatinederivate, sondern um enzymatisch hydrolysierte wasserlösliche bzw. in Wasser homogene und langzeitstabil dispergierbare Kollagenfragmente. Je nach Größe der Fragmente sind die Kollagen-Hydrolysate in Wasser löslich oder dispergierbar. Sie wirken emulsionsstabilisierend und werden daher häufig in der Nahrungs- und Kosmetikindustrie zur Einstellung der Rheologie von flüssigen Zubereitungen verwendet.

Im Rahmen der vorliegenden Erfindung werden bevorzugt kurzkettige Kollagen-Hydrolysate, d. h. Kollagen-Hydrolysate mit einem geringen Molekulargewicht, verwendet, da diese besonders gut vom Körper aufgenommen und verwertet werden können.

Kollagen-Hydrolysate verbessern darüber hinaus die Nährstoffversorgung von Gelenken aufgrund der gut verwertbaren Kollagenpeptide und ermöglichen so einen Wiederaufbau von geschädigten Knorpelgeweben. Da die Knochenneubildung ebenfalls über eine Vorstufe des Knorpelaufbaus geht, kann davon ausgegangen werden, dass Kollagen-Hydrolysate ebenfalls die Knochenneubildung positiv unterstützen und somit der Osteopathie von Dialysepatienten entgegenwirken.

Im Rahmen der vorliegenden Erfindung wird bevorzugt Kollagen-Hydrolysat auf Basis von Rinder-Kollagen eingesetzt.

Durch die Verwendung von Kollagen-Hydrolysat ist die erfindungsgemäße Zusammensetzung auch für Allergiker geeignet, da Allergien gegenüber Kollagen nur äußerst selten auftreten. Die bislang gebräuchlichen Eiweißpräparate zur Eiweißsubstitution bei Niereninsuffizienz basieren auf Molkeneiweißisolat, was jedoch den Nachteil mit sich bringt, dass immer mehr Menschen an einer Milchunverträglichkeit leiden, z. B. in Form einer Laktoseintoleranz oder Milcheiweißallergie, und somit die Präparate auf Molkenbasis nicht verwenden können.

Bei der erfindungsgemäßen Zusammensetzung handelt es sich um ein hochkonzentriertes flüssiges Eiweißkonzentrat, welches einerseits gut dosierbar ist, andererseits jedoch möglichst wenig Flüssigkeit enthält, da insbesondere bei Patienten mit Niereninsuffizienz die aufgenommene Flüssigkeitsmenge gering gehalten werden muss.

Die erfindungsgemäße Zusammensetzung ist äußerst einfach anzuwenden, da sie bereits als gebrauchsfertige oral einzunehmende Zubereitung vorliegt und nicht erst umständlich durch Anrühren eines Pulvers mit Wasser bereitgestellt werden muss.

Der Begriff "Eiweiß" umfasst im Rahmen der vorliegenden Erfindung sowohl "Proteine" als auch "Peptide" sowie deren jeweilige Derivate oder Fragmente. Unter "Proteine" werden im Allgemeinen aus Aminosäuren aufgebaute biologische Makromoleküle verstanden. Von den "Peptiden" unterscheiden sich die Proteine in der Länge der Aminosäuresequenz bzw. -kette: Peptide besitzen eine eher kurze Abfolge von Aminosäurefrequenzen, während Proteine mehrere Zehntausend Aminosäuren aufweisen können. Moleküle mit weniger als 100 Aminosäuren werden üblicherweise als Peptide bezeichnet, während Moleküle mit längeren Aminosäuresequenzen als Protein bezeichnet werden, wobei diese Unterscheidung nicht starr ist, sondern die Begriffe in Abhängigkeit von den jeweiligen Umständen entsprechend gewählt werden.

Im Rahmen der vorliegenden Erfindung hat es sich bewährt, wenn die Zusammensetzung die Kollagen-Hydrolysate in einer Konzentration von 25 bis 40 Gew.-%, insbesondere 30 bis 35 Gew.-%, bezogen auf die Zusammensetzung, enthält. Bei der erfindungsgemäßen Zusammensetzung handelt es sich somit um ein hochkonzentriertes Eiweißkonzentrat, welches eine effektive Zufuhr von Eiweißen, insbesondere von kurzkettigen Aminosäuresequenzen, d. h. Peptiden, ermöglicht ohne dass die Dialysepatienten größere Mengen Flüssigkeit aufnehmen müssten.

Im Allgemeinen handelt es sich bei der Zusammensetzung um eine wässrige Lösung oder Dispersion der Kollagen-Hydrolysate. Die erfindungsgemäße Zusammensetzung ist bevorzugt eine wässrige Dispersion oder Lösung von Kollagen-Hydrolysaten, welche optional noch weitere Stoffe, insbesondere Hilfs- und Zusatzstoffe, enthalten kann. Durch das Vorliegen der erfindungsgemäßen Zusammensetzung in flüssiger Form, insbesondere in Form einer Lösung oder Dispersion, ist die Compliance gegenüber bisher am Markt befindlichen Produkten deutlich erhöht, da die erfindungsgemäße Zusammensetzung zum einen sehr einfach zu dosieren ist und zum anderen der Patient bzw. Verbraucher durch die bereits gebrauchsfertig vorliegende Lösung oder Dispersion ein stets gleichbleibendes Produkt vor sich hat, dessen sensorische Eigenschaften stets gleich sind, d. h. das stets das gleiche Mundgefühl erzeugt und beispielsweise nicht klumpt.

Das Vorliegen in einer Dispersion oder Lösung der erfindungsgemäßen Zusammensetzung erhöht somit in erheblichem Maße die Akzeptanz bei Patienten und Verbrauchern.

Im Rahmen der vorliegenden Erfindung hat es sich bewährt, wenn die Zusammensetzung insbesondere zumindest im Wesentlichen ausschließlich Peptide, d. h. kurzkettige Eiweiße bzw. Aminosäuresequenzen, aus Kollagen-Hydrolysaten aufweist. Bei Peptiden handelt es sich wie bei Proteinen um Eiweiße, d. h. Aminosäuresequenzen bzw. -ketten, welche - wie zuvor ausgeführt - anhand ihrer Molekülmasse bzw. der Anzahl der verknüpften Aminosäuren unterschieden werden.

Im Rahmen der vorliegenden Erfindung weist die Zusammensetzung vorzugsweise nur Peptide aus Kollagen-Hydrolysaten, insbesondere auf Basis von Rinderkollagen, auf, da auf diese Weise einerseits eine hervorragende Verwertbarkeit der Peptide im Körper, insbesondere eine nahezu vollständige Resorption, gewährleistet wird und andererseits das Allergierisiko nahezu ausgeschlossen werden kann.

Gemäß einer bevorzugten Ausführungsform der vorliegenden Erfindung enthält die Zusammensetzung Peptide in einer Konzentration von 15 bis 50 Gew.-%, insbesondere 20 bis 40 Gew.-%, vorzugsweise 25 bis 35 Gew.-%, bevorzugt 28 bis 32 Gew.-%, bezogen auf die Zusammensetzung.

Besonders gute Ergebnisse werden im Rahmen der vorliegenden Erfindung erhalten, wenn mindestens 90 %, insbesondere mindestens 95 %, vorzugsweise mindestens 99 %, der Peptide Molekulargewichte von weniger als 3.000 g/mol, insbesondere weniger als 2.500 g/mol, vorzugsweise weniger als 2.000 g/mol, aufweisen.

Gleichermaßen hat es sich bewährt, wenn mindestens 90 %, insbesondere mindestens 95 %, vorzugsweise mindestens 99 %, der Peptide Molekulargewichte im Bereich von 200 bis 3.000 g/mol, insbesondere von 300 bis 2.500 g/mol, vorzugsweise von 500 bis 2.000 g/mol, aufweisen. Die erfindungsgemäß eingesetzten kurzkettigen Peptide werden vom Körper in hervorragender Weise verdaut und schnell resorbiert. Die Verdauung und Zerkleinerung der Eiweiße beginnt bereits durch Enzyme im Speichel. Im Magen erfolgt anschließend die erste Hydrolyse mittels gastrischer Enzyme. Die derart zerkleinerten Peptidfragmente werden anschließend durch Zugabe von Pankreasenzymen in Tri- und Dipeptide sowie einzelne Aminosäuren gespalten, welche im Dünndarm durch die intestinalen Zellen, die sogenannten Enterozyten, resorbiert und anschließend ins Blut abgegeben werden können. Es hat sich herausgestellt, dass mehr als 90 % des erfindungsgemäß eingesetzten niedermolekularen Kollagen-Hydrolysats nach oraler Einnahme umgehend verdaut und anschließend resorbiert wird.

Im Rahmen der vorliegenden Erfindung werden besonders gute Ergebnisse erhalten, wenn die Peptide die Aminosäure Arginin in Mengen von 4 bis 20 Gew.-%, insbesondere 5 bis 15 Gew.-%, vorzugsweise 6 bis 12 Gew.-%, bezogen auf das Gesamtgewicht der Peptide, enthalten.

Gleichfalls kann es vorgesehen sein, dass die Peptide die Aminosäure Glycin in Mengen von 10 bis 30 Gew.-%, insbesondere 15 bis 25 Gew.-%, vorzugsweise 18 bis 23 Gew.-%, bezogen auf das Gesamtgewicht der Peptide, enthalten.

Im Rahmen der vorliegenden Erfindung hat es sich darüber hinaus als vorteilhaft erwiesen, wenn die Peptide die Aminosäure Prolin in Mengen von 8 bis 25 Gew.-%, insbesondere 10 bis 20 Gew.-%, vorzugsweise 10 bis 15 Gew.-%, bezogen auf das Gesamtgewicht der Peptide, enthalten.

Gleichermaßen werden gute Ergebnisse erhalten, wenn die Peptide die Aminosäure Hydroxyprolin in Mengen von 10 bis 30 Gew.-%, insbesondere 15 bis 25 Gew.-%, vorzugsweise 18 bis 23 Gew.-%, bezogen auf das Gesamtgewicht der Peptide, enthalten.

Kollagen-Hydrolysate, welche die vorgenannten Aminosäuren in den angegebenen Mengen enthalten, werden besonders gut vom Körper resorbiert. Darüber hinaus ist die spezielle Zusammensetzung der erfindungsgemäß eingesetzten Peptide, insbesondere auf Basis von Kollagen-Hydrolysaten, besonders vorteilhaft und kann vom Körper beispielsweise unmittelbar bei der Versorgung von Muskeln und Knochen eingesetzt werden.

Erfindungsgemäß kann es zudem vorgesehen sein, dass die Zusammensetzung zusätzlich Tryptophan, vorzugsweise in Form von L-Tryptophan, enthält. Tryptophan zählt zu den aromatischen Aminosäuren und kann vom menschlichen Körper nicht gebildet werden. Mit anderen Worten ist Tryptophan eine essenzielle Aminosäure und muss mit der Nahrung zugeführt werden.

Erfindungsgemäß kann es daher vorgesehen sein, dass die erfindungsgemäße Zusammensetzung Tryptophan in einer Menge von 0,001 Gew.-% bis 0,5 Gew.-%, insbesondere 0,01 Gew.-% bis 0,4 Gew.-%, vorzugsweise 0,05 Gew.-% bis 0,2 Gew.- %, bevorzugt 0,08 Gew.-% bis 0,15 Gew.-%, bezogen auf die Zusammensetzung, enthält.

Im Allgemeinen enthält die Zusammensetzung weniger als 100 ppm, insbesondere weniger als 50 ppm, vorzugsweise weniger als 30 ppm, bevorzugt weniger als 10 ppm, Phosphat, bezogen auf das Gewicht der Zusammensetzung. Bei "ppm" handelt es sich um "parts per million", d. h. die Anzahl der Teile, beispielsweise Gewichtsteile, auf 1 Million Gesamtteile.

Die erfindungsgemäße Zusammensetzung eignet sich somit in hervorragender Weise als phosphatarme Eiweißergänzung, insbesondere für Patienten mit Niereninsuffizienz. Die erfindungsgemäße Zusammensetzung wirkt der Hyperphosphatämie und daraus resultierenden Krankheiten, wie beispielsweise dem sekundären Hyperparathyreoidismus, entgegen.

Im Rahmen der vorliegenden Erfindung wird es bevorzugt, wenn die Zusammensetzung insbesondere zumindest im Wesentlichen phosphatfrei ist.

Darüber hinaus ist es im Rahmen der vorliegenden Erfindung bevorzugt, wenn die Zusammensetzung einen gewichtsbezogenen Phosphor-Eiweiß-Quotienten (PEQ) im Bereich von 0,01 bis 5, insbesondere von 0,02 bis 2, vorzugsweise von 0,05 bis 1, bevorzugt von 0,08 bis 0,5, aufweist. Der Phosphor-Eiweiß-Quotient gibt an, wie viel Milligramm Phosphor, berechnet als Phosphat, die Zusammensetzung in Bezug auf in der Zusammensetzung enthaltenes Eiweiß in Gramm enthält. Der Phosphor-Eiweiß-Quotient ist für die Ernährung von Patienten mit Niereninsuffizienz, insbesondere von Dialysepatienten, bedeutend, wobei Lebensmittel bzw. Nahrungsergänzungsmittel mit einem besonders niedrigen Phosphor-Eiweiß-Quotienten zu bevorzugen sind. Die erfindungsgemäße Zusammensetzung weist einen äußerst geringen Phosphor-Eiweiß-Quotienten (PEQ) auf und ist damit in hervorragender Weise zur Substitution des erhöhten Eiweißbedarfs von Patienten mit chronischer Niereninsuffizienz, insbesondere von Dialysepatienten, geeignet.

Darüber hinaus kann die Zusammensetzung Kalium enthalten. Was nun die Menge an Kalium in der erfindungsgemäßen Zusammensetzung anbelangt, so kann diese in weiten Bereichen variieren. Besonders gute Ergebnisse werden jedoch im Rahmen der vorliegenden Erfindung erhalten, wenn die Zusammensetzung weniger als 200 ppm, insbesondere weniger als 100 ppm, vorzugsweise weniger als 50 ppm, bevorzugt weniger als 30 ppm, Kalium, bezogen auf das Gewicht der Zusammensetzung, enthält. Die erfindungsgemäße Zusammensetzung wirkt somit einem erhöhten Kaliumspiegel im Blutserum entgegen und verhindert somit einen zu hohen Kaliumspiegel im Blut, die sogenannte Hyperkaliämie.

Gleichfalls kann es im Rahmen der vorliegenden Erfindung vorgesehen sein, dass die Zusammensetzung Natrium enthält. Was nun wiederum die Menge an Natrium in der erfindungsgemäßen Zusammensetzung anbelangt, so kann diese gleichfalls in weiten Bereichen variieren. Besonders gute Ergebnisse werden jedoch im Rahmen der vorliegenden Erfindung erhalten, wenn die Zusammensetzung weniger als 1.000 ppm, insbesondere weniger als 800 ppm, vorzugsweise weniger als 600 ppm, Natrium, bezogen auf das Gewicht der Zusammensetzung, enthält. Die erfindungsgemäße Zusammensetzung ist somit nicht nur phosphat- und kaliumarm, sondern vielmehr auch natriumarm.

Darüber hinaus ist die erfindungsgemäße Zusammensetzung im Allgemeinen niederkalorisch, d. h. sie hat einen nur geringen physiologischen Brennwert. Dabei hat es sich im Rahmen der vorliegenden Erfindung bewährt, wenn die Zusammensetzung einen physiologischen Brennwert von weniger als 1000 kJ, insbesondere weniger als 800 kJ, vorzugsweise weniger als 600 kJ, bezogen auf 100 g Zusammensetzung, besitzt. Die erfindungsgemäße Zusammensetzung ist folglich auch für Diabetiker geeignet und dient speziell der Deckung eines erhöhten Eiweißbedarfs, ohne dass weitere Nährstoffe, welche der Patient unter Umständen nicht benötigt, unkontrolliert zugeführt werden.

Im Allgemeinen weist die Zusammensetzung Hilfs- und/oder Geschmacksstoffe auf.

Was nun die Menge an Hilfs- bzw. Geschmacksstoffen in der erfindungsgemäßen Zusammensetzung anbelangt, so kann diese naturgemäß in weiten Bereichen variieren. Es sich jedoch bewährt, wenn die Zusammensetzung die Hilfs- und/oder Geschmacksstoffe in Mengen von 0,01 bis 15 Gew.-%, insbesondere 0,1 bis 10 Gew.-%, vorzugsweise 0,2 bis 5 Gew.-%, bezogen auf die Zusammensetzung, enthält.

Wenn die erfindungsgemäße Zusammensetzung Hilfs- und/oder Geschmacksstoffe enthält, so sind diese üblicherweise ausgewählt aus Zuckern, insbesondere Saccharose, Glucose und/oder Fructose, Zuckeraustauschstoffen, Süßungsmitteln, Säuerungsmitteln, Konservierungsstoffen, Aromen, Farbstoffen und Stabilisatoren sowie deren Mischungen und Kombinationen. Die zuvor genannten Hilfs- und Geschmacksstoffe werden insbesondere eingesetzt, um die Sensorik, insbesondere das Mundgefühl sowie die Konsistenz und den Geschmack der erfindungsgemäßen Zusammensetzung für den Patienten möglichst angenehm zu gestalten. Wenn Hilfs- und/oder Geschmacksstoffe im Rahmen der vorliegenden Erfindung eingesetzt werden, so werden diese üblicherweise nur in äußerst geringen Mengen verwendet.

Gemäß einer bevorzugten Ausführungsform der vorliegenden Erfindung liegt die Zusammensetzung in Form einer wässrigen Lösung oder Dispersion vor, welche
(a) 20 bis 50 Gew.-% Kollagen-Hydrolysate, bezogen auf die Zusammensetzung,
(b) 0,1 bis 5 Gew.-% 3-Hydroxy-3-methylbuttersäure, und
(c) 0,01 bis 15 Gew.-% Hilfs- und/oder Geschmacksstoffe, jeweils bezogen auf die Zusammensetzung,
aufweist.

Gemäß einer weiteren, gleichfalls bevorzugten Ausführungsform der vorliegenden Erfindung liegt die erfindungsgemäße Zusammensetzung in Form einer wässrigen Lösung oder Dispersion vor und besteht aus
(a) 20 bis 50 Gew.-% Kollagen-Hydrolysaten, bezogen auf die Zusammensetzung,
(b) 0,1 bis 5 Gew.-% 3-Hydroxy-3-methylbuttersäure,
(c) 0,01 bis 15 Gew.-% Hilfs- und/oder Geschmacksstoffen, jeweils bezogen auf die Zusammensetzung, und
(d) Wasser,
wobei die Zusammensetzung
(I) weniger als 100 ppm Phosphat und
(II) weniger als 200 ppm Kalium,

jeweils bezogen auf das Gewicht der Zusammensetzung, enthält und
wobei die Zusammensetzung einen gewichtsbezogenen Phosphor-Eiweiß-Quotienten (PEQ) im Bereich von 0,01 bis 5 aufweist.

Bei der erfindungsgemäßen Zusammensetzung handelt es sich somit bevorzugt um ein hochkonzentriertes flüssiges Eiweißpräparat auf Basis von Kollagen-Hydrolysaten, welches phosphat- und kaliumarm bzw. -frei ist und sich in hervorragender Weise zur Deckung des erhöhten Eiweißbedarfs von Patienten mit Niereninsuffizienz, insbesondere von Dialysepatienten, eignet.

Wie zuvor bereits ausgeführt, liege die erfindungsgemäße Zusammensetzung üblicherweise in Form einer flüssigen Lösung oder Dispersion vor. Was nun die dynamische Viskosität der Zusammensetzung anbelangt, so kann diese gleichfalls in weiten Bereichen variieren. Es hat sich jedoch im Rahmen der vorliegenden Erfindung bewährt, wenn die Zusammensetzung eine dynamische Viskosität von höchstens 70 mPas, insbesondere höchstens 60 mPas, vorzugsweise höchstens 50 mPas, bei 20 °C aufweist.

Gleichfalls kann es vorgesehen sein, dass die Zusammensetzung eine dynamische Viskosität im Bereich von 0,01 bis 70 mPas, insbesondere 0,1 bis 60 mPas, vorzugsweise 1 bis 50 mPas, bei 20 °C aufweist.

Die erfindungsgemäße Zusammensetzung ist somit eine niederviskose Flüssigkeit, welche sich einfach dosieren lässt und darüber hinaus problemlos und nahezu rückstandsfrei aus Behältnissen entfernen lässt. Dies ist beispielsweise bei der Verpackung der erfindungsgemäßen Zusammensetzung in speziellen Dosierformen von Bedeutung, da sichergestellt sein muss, dass die tatsächlich in ein Behältnis gefüllte Menge an Zusammensetzung auch vom Verbraucher bzw. Patienten entnommen werden kann.

Weiterer Gegenstand der vorliegenden Erfindung - gemäß einem **zweiten** Aspekt der vorliegenden Erfindung - ist die zuvor beschriebene Zusammensetzung zur Verwendung bei der therapeutischen Behandlung von Eiweißmangelerkrankungen bei Patienten mit Niereninsuffizienz, insbesondere von Dialysepatienten.

Wie zuvor ausgeführt, eignet sich die erfindungsgemäße Zusammensetzung in hervorragender Weise zur Behandlung von Eiweißmangelerkrankungen, insbesondere auch als Nahrungsergänzungsmittel zur Deckung des erhöhten Eiweißbedarfs von Personen mit Niereninsuffizienz, insbesondere von Dialysepatienten.

Unter einer therapeutischen Behandlung ist dabei im Rahmen der vorliegenden Erfindung sowohl eine kurative Behandlung von Krankheiten bzw. Krankheitssymptomen zu verstehen als auch eine präventive Behandlung, d. h. eine Verhinderung des Ausbruchs von Krankheiten bzw. Krankheitssymptomen.

Weiterer Gegenstand der vorliegenden Erfindung - gemäß einem **dritten** Aspekt der vorliegenden Erfindung - ist die zuvor beschriebene Zusammensetzung zur Verwendung bei der therapeutischen Behandlung von Hyperphosphatämie, insbesondere bei Personen mit Niereninsuffizienz, beispielsweise Dialysepatienten.

Wiederum weiterer Gegenstand der vorliegenden Erfindung - gemäß einem **vierten** Aspekt der vorliegenden Erfindung - ist die erfindungsgemäße Zusammensetzung zur Verwendung bei der therapeutischen Behandlung des sekundären Hyperparathyreoidismus, insbesondere bei Personen mit Niereninsuffizienz, wie beispielsweise Dialysepatienten.

Durch die hohe Eiweißkonzentration bei gleichzeitig niedrigem Phosphatgehalt kann die erfindungsgemäße Zusammensetzung bei der Prävention und Behandlung der Phosphatämie und des daraus resultierenden sekundären Hyperparathyreoidismus eingesetzt werden.

Gemäß einer besonders bevorzugten Ausführungsform der vorliegenden Erfindung liegt die erfindungsgemäße Zusammensetzung zur Verwendung bei therapeutischen Behandlungen - insbesondere nach dem zweiten bis vierten Aspekt der vorliegenden Erfindung - in Form einer Einmaltagesdosis insbesondere zur oralen Einnahme vor. Die erfindungsgemäße Zusammensetzung wird somit bevorzugt in Form einer Einmaltagesdosis, eines sogenannten "*Shots*", bereitgestellt. Hierdurch wird dem Patienten nicht nur das aufwendige und fehleranfällige Anrühren eines pulverförmigen Eiweißkonzentrates mit einer Flüssigkeit erspart, sondern durch die Bereitstellung in Form einer Einmaltagesdosis werden auch Fehldosierungen vermieden.

Was nun die Menge an Peptiden in der Einmaltagesdosis anbelangt, so kann diese in weiten Bereichen variieren. Aus medizinischer Sicht ist es jedoch vorteilhaft, wenn die Einmaltagesdosis die Peptide in einer Menge von 10 bis 30 g, insbesondere 15 bis 25 g, vorzugsweise 18 bis 22 g, bevorzugt genau 20 g, bezogen auf die Einmaltagesdosis, enthält. Die zuvor genannten Werte entsprechen der Deckungslücke an Eiweiß bei Patienten mit chronischer Niereninsuffizienz, insbesondere von Dialysepatienten, welche nicht mehr als 1.000 mg Phosphat am Tag zu sich nehmen sollen.

Darüber hinaus hat es sich im Rahmen der vorliegenden Erfindung gleichfalls bewährt, wenn die Einmaltagesdosis ein Volumen von 30 bis 120 ml, insbesondere 40 bis 100 ml, vorzugsweise 50 bis 80 ml, bevorzugt 60 bis 70 ml, aufweist. Bei der erfindungsgemäßen Zusammensetzung in Form einer Einmaltagesdosis handelt es sich somit um ein hochkonzentriertes flüssiges Eiweißpräparat, welches keine unnötige und übermäßige Flüssigkeitszufuhr für Dialysepatienten mit sich bringt.

Üblicherweise ist die Einmaltagesdosis gleichfalls sehr niederkalorisch, insbesondere weist die Einmaltagesdosis einen physiologischen Brennwert von weniger als 600 kJ, insbesondere weniger als 500 kJ, vorzugsweise weniger als 450 kJ, bezogen auf die Einmal-Tagesdosis, auf. Die Eimaltagesdosis zur therapeutischen Behandlung kann somit problemlos von Diabetikern verwendet werden.

Bei der erfindungsgemäßen Zusammensetzung handelt es sich somit um ein hochkonzentriertes, flüssiges phosphat- und kaliumarmes Eiweißkonzentrat, welches sich in hervorragender Weise zur Substitution des erhöhten Eiweißbedarfs von Patienten mit Niereninsuffizienz, insbesondere von Dialysepatienten eignet.

Die erfindungsgemäße Zusammensetzung ist auf Basis von Kollagen-Hydrolysaten ausgebildet, wodurch zum einen ein besonders vorteilhaftes und für den Körper essentielles Aminosäurespektrum bereitgestellt wird und andererseits die Gefahr allergischer Reaktionen weitestgehend ausgeschlossen wird.

Die im Rahmen der erfindungsgemäßen Zusammensetzung verwendeten Kollagen-Hydrolysate haben ein äußerst geringes Molekulargewicht und werden folglich vom Körper besonders leicht verdaut und nahezu vollständig resorbiert.

Die erfindungsgemäße Zusammensetzung in Form einer Einmaltagesdosis ist genau an den erhöhten Eiweißbedarf von Dialysepatienten angepasst.

Die Verwendung von Kollagen-Hydrolysat wirkt in spezifischer Weise auch der bei Dialysepatienten oftmals auftretenden Osteopathie entgegen.

Durch die verbesserte Eiweißaufnahme sowie die geringe Phosphatlast der erfindungsgemäßen Zusammensetzung kann die Mortalität von Dialysepatienten reduziert werden.

Die erfindungsgemäße Zusammensetzung ist ferner auch für Diabetiker geeignet, da sie keinen zusätzlichen physiologischen Brennwert in Form von Kohlenhydraten enthält.

Die erfindungsgemäße Zusammensetzung wird in Form einer gebrauchsfertigen Einmaltagesdosis bereitgestellt, d. h. es entfällt das bei Eiweißpräparaten des Standes der Technik übliche Anrühren von Pulvern, welches die Gefahr von Fehldosierungen mit sich bringt und beispielsweise von älteren Menschen nicht mehr eigenständig durchgeführt werden kann.

Die erfindungsgemäße Zusammensetzung ist aufgrund der Bereitstellung in flüssiger Form besonders einfach anzuwenden und zu dosieren.

Für weitergehende Einzelheiten zu der erfindungsgemäßen Zusammensetzung zur Verwendung bei therapeutischen Behandlungen kann auf die vorherigen Ausführungen zur erfindungsgemäßen Zusammensetzung verwiesen werden, welche in Bezug auf die erfindungsgemäße Zusammensetzung zur Verwendung bei therapeutischen Behandlungen entsprechend gelten.

Wiederum weiterer Gegenstand der vorliegenden Erfindung - gemäß einem **fünften** Aspekt der vorliegenden Erfindung - ist die Verwendung einer zuvor beschriebenen Zusammensetzung als Nahrungsergänzungsmittel.

Insbesondere kann die zuvor beschriebene Zusammensetzung zur Substitution des erhöhten Eiweißbedarfs bei Niereninsuffizienz, insbesondere von Dialysepatienten, verwendet werden.

Für weitere Einzelheiten zu der erfindungsgemäßen Verwendung kann auf die vorherigen Erfindungsaspekte verwiesen werden, welche in Bezug auf die erfindungsgemäße Verwendung entsprechend gelten.

Wiederum weiterer Gegenstand der vorliegenden Erfindung - gemäß einem **sechsten** Aspekt der vorliegenden Erfindung - ist die Verwendung einer zuvor beschriebenen Zusammensetzung bei der therapeutischen Behandlung von Eiweißmangelerkrankungen bei Personen mit Niereninsuffizienz, insbesondere von Dialysepatienten.

Für weitere Einzelheiten zu diesem Erfindungsaspekt kann auf die vorherigen Ausführungen zu den übrigen Erfindungsaspekten verwiesen werden, welche in Bezug auf die erfindungsgemäße Verwendung entsprechend gelten.

Wiederum weiterer Gegenstand der vorliegenden Erfindung - gemäß einem **siebten** Aspekt der vorliegenden Erfindung - ist die Verwendung einer zuvor beschriebenen Zusammensetzung bei der therapeutischen Behandlung von Hyperphosphatämie, insbesondere bei Personen mit Niereninsuffizienz, wie beispielsweise Dialysepatienten.

Für weitergehende Einzelheiten zu diesem Erfindungsaspekt kann auf die Ausführungen zu den übrigen Erfindungsaspekten verwiesen werden, welche in Bezug auf die erfindungsgemäße Verwendung entsprechend gelten.

Schließlich ist wiederum weiterer Gegenstand der vorliegenden Erfindung - gemäß einem **achten** Aspekt der vorliegenden Erfindung - die Verwendung einer zuvor beschriebenen Zusammensetzung bei der therapeutischen Behandlung des sekundären Hyperparathyreoidismus, insbesondere bei Personen mit Niereninsuffizienz, beispielsweise von Dialysepatienten.

Was nun die erfindungsgemäßen Verwendungen anbelangt, so ist es bevorzugt, wenn die Zusammensetzung in Form eine Einmaltagesdosis insbesondere zur oralen Einnahme, insbesondere als sogenannter "*Shot*", vorliegt.

Gleichfalls werden im Rahmen der vorliegenden Erfindung besonders gute Ergebnisse erhalten, wenn die Einmaltagesdosis die Peptide in einer Menge von 10 bis 30 g, insbesondere 15 bis 25 g, vorzugsweise 18 bis 22 g, bevorzugt 20 g, bezogen auf die Einmaltagesdosis, enthält.

Wenn die Zusammensetzung in Form einer Einmaltagesdosis verwendet wird, so weist die Einmaltagesdosis ein Volumen von 30 bis 120 ml, insbesondere 40 bis 100 ml, vorzugsweise 50 bis 80 ml, bevorzugt 60 bis 70 ml, auf.

Darüber hinaus weist die im Rahmen der vorliegenden Erfindung verwendete Zusammensetzung nur einen äußerst geringen physiologischen Brennwert auf, ist folglich niederkalorisch. Was nun den physiologischen Brennwert der Einmaltagesdosis anbelangt, so kann dieser in weiten Bereichen variieren. Im Rahmen der vorliegenden Erfindung werden jedoch besonders gute Ergebnisse erhalten, wenn der physiologische Brennweit der Einmaltagesdosis weniger als 600 kJ, insbesondere weniger als 500 kJ, vorzugsweise weniger als 450 kJ, bezogen auf die Einmaltagesdosis, beträgt.

Für weitergehende Einzelheiten zu den Erfindungsaspekten betreffend die erfindungsgemäßen Verwendungen kann auf die vorherigen Ausführungen zu den übrigen Erfindungsaspekten verwiesen werden, welche in Bezug auf die erfindungsgemäßen Verwendungen entsprechend gelten.

Der Gegenstand der vorliegenden Erfindung wird nachfolgend durch die Ausführungsbeispiele verdeutlicht, welche den Gegenstand der vorliegenden Erfindung exemplarisch verdeutlichen, ohne in irgendeiner Form einschränkend zu wirken.

### Ausführungsbeispiele:

Aus Wasser, Kollagen-Hydrolysat, Zitronensäure als Säuerungsmittel, Fructose, Kaliumsorbat und Natriumbenzoat als Konservierungsstoffen, Sucralose als Süßungsmittel und Aromastoffen werden niedrigviskose Dispersionen angerührt.

Die Dispersionen sind homogen und über Monate lagerstabil. Sie werden in Portionen von 60 ml mit einem Eiweißgehalt von 18 g bzw. 20 g abgepackt, was einer Einmaltagesdosis, einem sogenannten "*Shot*" entspricht.

Die Zusammensetzungen der Einmaltagesdosen sind in den nachfolgenden Tabellen angegeben.

**Tabelle 1: Nährwertangaben bezüglich einer Einmaltagesdosis von 60 ml**

| | | **ml** | | **pro Portion 60** |
|---|---|---|---|---|
| Brennwert | | kJ | | 350 |
| | | kcal | | 82 |
| Fett davon gesättigte Fettsäuren | | g | | 0 |
| | | g | | 0 |
| Kohlenhydrate davon Zucker | | g | | 1,2 |
| | | g | | 1,2 |
| Eiweiß | | g | | 18,0 |
| Salz | | 9 | | 0,3 |
| Nährstoffe: | | | | |
| HMB | | mg | | 480 |
| Kollagenhydrolysat¹ davon Aminosäuren: | | mg | | 20.000 |
| Valin | | mg | | 432 |
| Isoleucin | | mg | | 270 |
| Leucin | | mg | | 522 |
| Lysin | | mg | | 612 |
| Methionin | | mg | | 108 |
| Threonin | | mg | | 342 |
| Phenylalanin | | mg | | 378 |
| Arginin | | mg | | 1.512 |
| Histidin | | mg | | 144 |
| Tyrosin | | mg | | 90 |
| Prolin | | mg | | 2.070 |
| Alanin | | mg | | 1.458 |
| Asparaginsäure | | mg | | 1.188 |
| Serin | | mg | | 612 |
| Glutaminsäure | | mg | | 2.232 |
| Glycin | | mg | | 3.708 |
| Hydroxylysin | | mg | | 216 |
| Hydroxyprolin | | mg | | 2.052 |

| | | | | |
|---|---|---|---|---|
| 1 90 Gew.-% der Peptide des eingesetzten Kollagen-Hydrolysats besitzen ein Molekulargewicht von weniger als 2.000 g/mol | | | | |

**Tabelle 2: Nährwertangaben bezüglich einer weiteren Einmaltagesdosis von 60 ml**

| | | **ml** | | **pro Portion 60** |
|---|---|---|---|---|
| Brennwert | | kJ | | 403 |
| | | kcal | | 95 |
| Fett davon gesättigte Fettsäuren | | g | | <0,5 |
| | | g | | <0,1 |
| Kohlenhydrate davon Zucker | | g | | 1,4 |
| | | g | | 1,3 |
| Eiweiß | | g | | 20,0 |
| Salz | | g | | 0,14 |
| Nährstoffe: | | | | |
| HMB | | mg | | 250 |
| Tryptophan | | mg | | 60 |
| Kollagenhydrolysat¹ davon Aminosäuren: | | mg | | 22.000 |
| Valin | | mg | | 480 |
| Isoleucin | | mg | | 300 |
| Leucin | | mg | | 570 |
| Lysin | | mg | | 670 |
| Methionin | | mg | | 120 |
| Threonin | | mg | | 380 |
| Phenylalanin | | mg | | 420 |
| Arginin | | mg | | 1.660 |
| Histidin | | mg | | 160 |
| Tyrosin | | mg | | 100 |
| Prolin | | mg | | 2.280 |
| Alanin | | mg | | 1.600 |
| Asparaginsäure | | mg | | 1.310 |
| Serin | | mg | | 670 |
| Glutaminsäure | | mg | | 2.460 |
| Glycin | | mg | | 4.080 |
| Hydroxylysin | | mg | | 240 |
| Hydroxyprolin | | mg | | 2.260 |

| | | | | |
|---|---|---|---|---|
| ¹ 90 Gew.-% der Peptide des eingesetzten Kollagen-Hydrolysats besitzen ein Molekulargewicht von weniger als 2.000 g/mol | | | | |

## Patentansprüche

1. Zusammensetzung, insbesondere zur Eiweißsubstitution bei Niereninsuffizienz,
**dadurch gekennzeichnet,**
**dass** die Zusammensetzung in flüssiger Form vorliegt und
(a) Kollagen-Hydrolysate in einer Menge von 20 bis 50 Gew.-%, und
(b) 3-Hydroxy-3-methylbuttersäure in einer Menge von 0,1 bis 5 Gew.-%,
jeweils bezogen auf die Zusammensetzung, enthält.

2. Zusammensetzung nach Anspruch 1, **dadurch gekennzeichnet,**
**dass** die Zusammensetzung 3-Hydroxy-3-methylbuttersäure in einer Menge von 0,2 bis 3 Gew.-%, insbeondere 0,3 bis 2 Gew.-%, vorzugsweise 0,4 bis 1,5 Gew.-%, bevorzugt 0,4 bis 1 Gew.-%, bezogen auf die Zusammensetzung, enthält; und/oder
**dass** die Zusammensetzung die Kollagen-Hydrolysate in einer Konzentration von 25 bis 40 Gew.-%, insbesondere 30 bis 35 Gew.-%, bezogen auf die Zusammensetzung, enthält.

3. Zusammensetzung nach einem der vorangehenden Ansprüche, **dadurch gekennzeichnet,**
**dass** die Zusammensetzung insbesondere im Wesentlichen ausschließlich Peptide aus Kollagen-Hydrolysaten aufweist; und/oder
**dass** die Zusammensetzung Peptide in einer Konzentration von 15 bis 50 Gew.-%, insbesondere 20 bis 40 Gew.-%, vorzugsweise 25 bis 35 Gew.-%, bevorzugt 28 bis 32 Gew.-%, bezogen auf die Zusammensetzung, enthält.

4. Zusammensetzung nach einem der vorangehenden Ansprüche, **dadurch gekennzeichnet,**
**dass** mindestens 90 %, insbesondere mindestens 95 %, vorzugsweise mindestens 99 %, der Peptide Molekulargewichte von weniger als 3.000 g/mol, insbesondere weniger als 2.500 g/mol, vorzugsweise weniger als 2.000 g/mol, aufweisen und/oder
**dass** mindestens 90 %, insbesondere mindestens 95 %, vorzugsweise mindestens 99 %, der Peptide Molekulargewichte im Bereich von 200 bis 3.000 g/mol, insbesondere von 300 bis 2.500 g/mol, vorzugsweise 500 bis 2.000 g/mol, aufweisen.

5. Zusammensetzung nach einem der vorangehenden Ansprüche, **dadurch gekennzeichnet,**
**dass** die Peptide die Aminosäure Arginin in Mengen von 4 bis 20 Gew.-%, insbesondere 5 bis 15 Gew.-%, vorzugsweise 6 bis 12 Gew.-%, bezogen auf das Gesamtgewicht der Peptide, enthalten und/oder
**dass** die Peptide die Aminosäure Glycin in Mengen von 10 bis 30 Gew.-%, insbesondere 15 bis 25 Gew.-%, vorzugsweise 18 bis 23 Gew.-%, bezogen auf das Gesamtgewicht der Peptide, enthalten und/oder
**dass** die Peptide die Aminosäure Prolin in Mengen von 8 bis 25 Gew.-%, insbesondere 10 bis 20 Gew.-%, vorzugsweise 10 bis 15 Gew.-%, bezogen auf das Gesamtgewicht der Peptide, enthalten und/oder
die Peptide die Aminosäure Hydroxyprolin in Mengen von 10 bis 30 Gew.-%, insbesondere 15 bis 25 Gew.-%, vorzugsweise 18 bis 23 Gew.-%, bezogen auf das Gesamtgewicht der Peptide, enthalten.

6. Zusammensetzung nach einem der vorangehenden Ansprüche, **dadurch gekennzeichnet, dass** die Zusammensetzung weniger als 100 ppm, insbesondere weniger als 50 ppm, vorzugsweise weniger als 30 ppm, bevorzugt weniger als 10 ppm, Phosphat, bezogen auf das Gewicht der Zusammensetzung, enthält, inbesondere wobei die Zusammensetzung zumindest im Wesentlichen phosphatfrei ist.

7. Zusammensetzung nach einem der vorangehenden Ansprüche, **dadurch gekennzeichnet, dass** die Zusammensetzung einen gewichtsbezogenen Phosphor-Eiweiß-Quotienten (PEQ) im Bereich von 0,01 bis 5, insbesondere von 0,02 bis 2, vorzugsweise von 0,05 bis 1, bevorzugt von 0,08 bis 0,5, aufweist.

8. Zusammensetzung nach einem der vorangehenden Ansprüche, **dadurch gekennzeichnet,**
**dass** die Zusammensetzung weniger als 200 ppm, insbesondere weniger als 100 ppm, vorzugsweise weniger als 50 ppm, bevorzugt weniger als 30 ppm, Kalium, bezogen auf das Gewicht der Zusammensetzung, enthält; und/oder
**dass** die Zusammensetzung weniger als 1.000 ppm, insbesondere weniger als 800 ppm, vorzugsweise weniger als 600 ppm, Natrium, bezogen auf das Gewicht der Zusammensetzung, enthält.

9. Zusammensetzung nach einem der vorangehenden Ansprüche, **dadurch gekennzeichnet, dass** die Zusammensetzung einen physiologischen Brennwert von weniger als 1000 kJ, insbesondere weniger als 800 kJ, vorzugsweise weniger als 600 kJ, bezogen auf 100 g Zusammensetzung, besitzt.

10. Zusammensetzung nach einem der vorangehenden Ansprüche, **dadurch gekennzeichnet, dass** die Zusammensetzung Hilfs- und/oder Geschmacksstoffe enthält,
insbesondere wobei die Zusammensetzung die Hilfs- und/oder Geschmacksstoffe in Mengen von 0,01 bis 15 Gew.-%, insbesondere 0,1 bis 10 Gew.-%, vorzugsweise 0,2 bis 5 Gew.-%, bezogen auf die Zusammensetzung, enthält, und/oder
insbesondere wobei die Hilfs- und/oder Geschmacksstoffe ausgewählt sind aus Zuckern, insbesondere Saccharose, Glucose und/oder Fructose, Zuckeraustauschstoffen, Süßungsmitteln, Säuerungsmitteln, Konservierungsstoffen, Aromen, Farbstoffen und Stabilisatoren sowie deren Mischungen und Kombinationen.

11. Zusammensetzung nach einem der vorangehenden Ansprüche in Form einer wässrigen Lösung oder Dispersion, bestehend aus
(a) 20 bis 50 Gew.-% Kollagen-Hydrolysaten, bezogen auf die Zusammensetzung,
(b) 0,1 bis 5 Gew.-% 3-Hydroxy-3-methylbuttersäure,
(c) 0,01 bis 15 Gew.-% Hilfs- und/oder Geschmacksstoffen, jeweils bezogen auf die Zusammensetzung, und
(d) Wasser,
wobei die Zusammensetzung
(I) weniger als 100 ppm Phosphat und
(II) weniger als 200 ppm Kalium,
jeweils bezogen auf das Gewicht der Zusammensetzung, enthält und
wobei die Zusammensetzung einen gewichtsbezogenen Phosphor-Eiweiß-Quotienten (PEQ) im Bereich von 0,01 bis 5 aufweist.

12. Zusammensetzung nach einem der vorangehenden Ansprüche zur Verwendung bei der therapeutischen Behandlung von Eiweißmangelerkrankungen bei Personen mit Niereninsuffizienz, insbesondere von Dialysepatienten.

13. Zusammensetzung nach einem der vorangehenden Ansprüche zur Verwendung bei der therapeutischen Behandlung von Hyperphosphatämie, insbesondere bei Personen mit Niereninsuffizienz.

14. Zusammensetzung nach einem der vorangehenden Ansprüche zur Verwendung bei der therapeutischen Behandlung des sekundären Hyperparathyreoidismus, insbesondere bei Personen mit Niereninsuffizienz.

15. Verwendung einer Zusammensetzung nach einem der Ansprüche 1 bis 11, als Nahrungsergänzungsmittel.
